# EUROPEAN PATENT APPLICATION

(11) **EP 4 775 962 A1**
(43) Date of publication of application: **15.07.2026**
(21) Application number: 26150035.9
(22) Date of filing: 02.01.2026
(51) Int. Cl.: G01N 21/552, G01N 21/05, G01N 21/85, G02B 6/12

(54) **ENHANCED RING RESONATOR DUAL FREQUENCY COMB SPECTROSCOPY GAS SENSOR**

(30) Priority: 12.01.2025 US 202563744283 P; 12.12.2025 US 202519418568
(71) Applicant: Honeywell International Inc., Charlotte, NC 28202 (US)
(72) Inventor: FENG, Chen, Charlotte, 28202 (US); SHAFAI, Moin S., Charlotte, 28202 (US); BROWN, Andy Walker, Charlotte, 28202 (US); VENKATARAYALU, Suresh, Charlotte, 28202 (US)
(74) Representative: Haseltine Lake Kempner LLP

(57) **Abstract**

The present disclosure provides a photonic gas sensor chip comprising a substrate, a silicon oxide layer disposed on the substrate, a heating element disposed on the silicon oxide layer, a silicon oxide base disposed on the heating element, and a silicon nitride waveguide disposed on the silicon oxide base. The silicon nitride waveguide comprises an extended resonant ring waveguide comprising spiral waveguide sections. The spiral sections increase optical path length while maintaining compact footprint. The heating element enables thermal tuning of resonant frequency to match dual frequency comb spectroscopy repetition frequencies.

## Description

### CROSS-REFERENCE TO RELATED APPLICATIONS

This application claims priority to U.S. Provisional Patent Application No. 63/744,283, filed on January 12, 2025, which is incorporated herein by reference in its entirety.

### TECHNICAL FIELD

Embodiments of the present disclosure generally relate to gas spectroscopy, and, more particularly, to enhanced ring resonator dual frequency comb spectroscopy gas sensor.

### BACKGROUND

Applicant has identified many technical challenges associated with photonic ring resonators. Through applied effort, ingenuity, and innovation, many of these identified problems have been solved by developing solutions that are included in embodiments of the present disclosure, many examples of which are described in detail herein.

### SUMMARY

According to an aspect of the present disclosure, a photonic gas sensor chip is provided. The photonic gas sensor chip comprises a substrate. The photonic gas sensor chip comprises a silicon oxide layer disposed on the substrate. The photonic gas sensor chip comprises a heating element disposed on the silicon oxide layer. The photonic gas sensor chip comprises a silicon oxide base disposed on the heating element. The photonic gas sensor chip comprises a silicon nitride waveguide disposed on the silicon oxide base. The silicon nitride waveguide comprises an extended resonant ring waveguide comprising a plurality of spiral waveguide sections.

According to other aspects of the present disclosure, the photonic gas sensor chip may include one or more of the following features. The photonic gas sensor chip may further comprise a silicon oxide cover disposed on the silicon nitride waveguide. The plurality of spiral waveguide sections may comprise four waveguide sections. The plurality of spiral waveguide sections may be arranged at regular angular intervals around a central axis of the extended resonant ring waveguide. The silicon nitride waveguide may further comprise an input waveguide configured to couple light into the extended resonant ring waveguide and an output waveguide configured to light from the extended resonant ring waveguide. The extended resonant ring waveguide may be positioned between the input waveguide and the output waveguide. The silicon nitride waveguide may further comprise a light input coupler configured to couple light into the input waveguide and a light output coupler configured to extract light from the output waveguide. The light input coupler and the light output coupler may each comprise periodic grating features. Each of the input waveguide and output waveguide may be straight bus waveguide. The extended resonant ring waveguide may have a ring diameter of approximately 3mm and an effective optical path length of approximately 150mm. The extended resonant ring waveguide may be configured to resonate at approximately 2GHz to match a dual-frequency comb spectroscopy repetition frequency. The heating element may be an aluminum heating element having a spiral pattern. The heating element may comprise a heating current input pad configured to receive electrical current from an external power source for supplying the heating element and a heating current output pad electrically connected to the heating current input pad. The heating element may comprise heating coils arranged below the extended resonant ring waveguide, wherein the heating element is configured to match a resonant frequency of the extended resonant ring waveguide to a repetition frequency of an optical frequency comb.

According to another aspect of the present disclosure, a photonic gas sensing device is provided. The photonic gas sensing device comprises a housing defining a chamber. The photonic gas sensing device comprises a photonic gas sensor positioned within at least a portion of the housing. The photonic gas sensor comprises a silicon nitride waveguide having an extended resonant ring waveguide that extends an optical path length within the extended resonant ring waveguide. The photonic gas sensing device comprises a gas inlet configured for introducing gas into the chamber. The photonic gas sensing device comprises a light inlet configured to receive light from an external light source.

According to other aspects of the present disclosure, the photonic gas sensing device may include one or more of the following features. The photonic gas sensing device may further comprise a gas outlet configured for removing the gas from the chamber and a light outlet configured for outputting at least a portion of the light from the photonic gas sensor. The photonic gas sensor may further comprise a heating element disposed below the silicon nitride waveguide, wherein the heating element comprises heating coils arranged below the extended resonant ring waveguide. The extended resonant ring waveguide may comprise a plurality of spiral waveguide sections arranged at regular angular intervals around a central axis of the extended resonant ring waveguide.

According to another aspect of the present disclosure, a method of gas sensing using dual frequency comb spectroscopy is provided. The method comprises directing input light beam from a dual frequency comb into a silicon nitride waveguide positioned within a chamber, wherein the input light beam comprises a first optical frequency comb of a pair of optical frequency combs, wherein the silicon nitride waveguide comprises an extended resonant ring waveguide with spiral waveguide sections. The method comprises introducing a gas sample into the chamber, wherein the gas travels over the silicon nitride waveguide and interacts with an evanescent field of the silicon nitride waveguide. The method comprises combining output optical frequency comb corresponding to output light beam exiting the chamber and a second optical frequency comb from the pair of optical frequency combs to generate a combined optical frequency comb. The method comprises detecting, using an optical detector, the combined optical frequency comb. The method comprises processing the combined optical frequency comb to determine a presence or concentration of a target gas in the gas sample by performing one or more of signal processing or data processing based on the combined optical frequency comb.

According to other aspects of the present disclosure, the method may include one or more of the following features. A resonant frequency of the extended resonant ring waveguide may be matched to a repetition frequency of the first optical frequency comb using a heating element positioned below the silicon nitride waveguide.

The above summary is provided merely for purposes of summarizing some example embodiments to provide a basic understanding of some aspects of the present disclosure. Accordingly, it will be appreciated that the above-described embodiments are merely examples and should not be construed to narrow the scope or spirit of the disclosure in any way. It will be appreciated that the scope of the present disclosure encompasses many potential embodiments in addition to those here summarized, some of which will be further described below.

### BRIEF DESCRIPTION OF FIGURES

The description of the illustrative embodiments may be read in conjunction with the accompanying figures. It will be appreciated that, for simplicity and clarity of illustration, elements illustrated in the figures have not necessarily been drawn to scale, unless described otherwise. For example, the dimensions of some of the elements may be exaggerated relative to other elements, unless described otherwise. Embodiments incorporating teachings of the present disclosure are shown and described with respect to the figures presented herein, in which:
FIGS. 1A-1C illustrate various views of a photonic gas sensor chip in accordance with at least some example embodiments of the present disclosure.
FIGS. 2A-2C illustrate various views of an example waveguide with a ring resonator structure in accordance with at least some example embodiments of the present disclosure.
FIGS. 3A-3B illustrate various vies of a heating element in accordance with at least some example embodiments of the present disclosure.
FIGS. 4A-4D illustrate various views of a photonic gas sensor in accordance with at least some example embodiments of the present disclosure.
FIG. 4E illustrates a block diagram of a photonic gas sensing system in accordance with at least some example embodiments of the present disclosure.
FIG. 5 illustrates a block diagram of an example control device for gas spectroscopy system in accordance with at least some example embodiments of the present disclosure.

### DETAILED DESCRIPTION

Some embodiments of the present disclosure will now be described more fully hereinafter with reference to the accompanying drawings, in which some, but not all embodiments of the disclosure are shown. Indeed, these disclosures may be embodied in many different forms and should not be construed as limited to the embodiments set forth herein; rather, these embodiments are provided so that this disclosure will satisfy applicable legal requirements. Like numbers refer to like elements throughout.

As used herein, terms such as "front," "rear," "top," "bottom," "left," "right," etc. are used for explanatory purposes in the examples provided below to describe the relative position of certain components or portions of components. Furthermore, as would be evident to one of ordinary skill in the art in light of the present disclosure, the terms "substantially" and "approximately" indicate that the referenced element or associated description is accurate to within applicable engineering tolerances.

As used herein, the term "comprising" means including but not limited to and should be interpreted in the manner it is typically used in the patent context. Use of broader terms such as comprises, includes, and having should be understood to provide support for narrower terms such as consisting of, consisting essentially of, and comprised substantially of.

The phrases "in one embodiment," "according to one embodiment," "in some embodiments," and the like generally mean that the particular feature, structure, or characteristic following the phrase may be included in at least one embodiment of the present disclosure and may be included in more than one embodiment of the present disclosure (importantly, such phrases do not necessarily refer to the same embodiment).

The phrases "in one example," "according to one example," "in some examples," and the like generally mean that the particular feature, structure, or characteristic following the phrase may be included in at least one example of the present disclosure and may be included in more than one example of the present disclosure (importantly, such phrases do not necessarily refer to the same example).

If the specification states a component or feature "may," "can," "could," "should," "would," "preferably," "possibly," "typically," "optionally," "for example," "as an example," "in some examples," "often," or "might" (or other such language) be included or have a characteristic, that specific component or feature is not required to be included or to have the characteristic. Such component or feature may be optionally included in some examples, or it may be excluded.

The word "example" or "exemplary" is used herein to mean "serving as an example, instance, or illustration." Any implementation described herein as "example" or "exemplary" is not necessarily to be construed as preferred or advantageous over other implementations.

The term "electrically connected," "electronically coupled," "electronically coupling," "electronically couple," "in communication with," "in electronic communication with," or "connected" in the present disclosure refers to two or more elements or components being connected through wired means and/or wireless means, such that signals, electrical voltage/current, data and/or information may be transmitted to and/or received from these elements or components.

The term "component" may refer to an article, a device, or an apparatus that may comprise one or more surfaces, portions, layers and/or elements. For example, an example component may comprise one or more substrates that may provide underlying layer(s) for the component and may comprise one or more elements that may form part of and/or are disposed on top of the substrate. The term "element" may refer to an article, a device, or an apparatus that may provide one or more functionalities.

### Overview

Conventional photonic ring resonators face technical challenges in gas spectroscopy applications, particularly when attempting to integrate with dual frequency comb spectroscopy (DCS) systems. The resonant frequency of traditional photonic ring resonators may be too high to effectively match the repetition frequency of DCS systems due to the physical constraints imposed by the feature size of the ring resonator structure. While increasing the size of the resonator can potentially match DCS frequencies, oversized ring resonators may be limited by manufacturing difficulties and practical application constraints in chip-scale implementations.

Dual frequency comb spectroscopy represents an advanced optical technique for gas detection and analysis, offering high precision and sensitivity for identifying and quantifying gas concentrations. In various applications and context, the effectiveness of DCS systems depends on precise frequency matching between the optical resonator and the comb repetition frequency (e.g., DCS repetition frequency). When conventional ring resonators fail to achieve this frequency matching, the sensitivity and performance of the gas sensing system may be compromised.

The enhanced ring resonator technology disclosed herein addresses these technical challenges by incorporating spiral waveguide within the ring resonator structure. The spiral waveguide may increase the optical path length while maintaining a compact physical footprint, thereby reducing the resonant frequency to match DCS repetition frequencies. This approach may enable the development of chip-scale DCS gas sensor devices and systems with enhanced sensitivity and improved performance characteristics.

The spiral waveguide configuration may provide several technical advantages over conventional ring resonator designs. The extended optical path length created by the spiral sections may increase the interaction between light and gas molecules, potentially enhancing the sensitivity of gas detection. The compact design may allow for integration into miniaturized sensor packages suitable for various industrial applications, environmental applications, healthcare applications, or other applications.

In some examples, the enhanced ring resonator may incorporate thermal tuning capabilities to achieve precise frequency matching with DCS systems. Thermal tuning may allow for fine adjustment of the resonant frequency through controlled thermal expansion and thermal refractive index change of the waveguide material, enabling stable operation and maintaining optimal resonance conditions for enhanced gas sensing performance.

The enhanced ring resonator technology may enable the production of highly sensitive gas sensors capable of detecting low concentrations of target gases across various applications. Environmental monitoring systems may benefit from the enhanced sensitivity for detecting trace amounts of pollutants or greenhouse gases. Industrial process control applications may utilize the technology for monitoring gas concentrations in manufacturing environments. Healthcare applications may leverage the enhanced sensitivity for breath analysis and medical diagnostics.

### Example Photonic Gas Sensor Chip

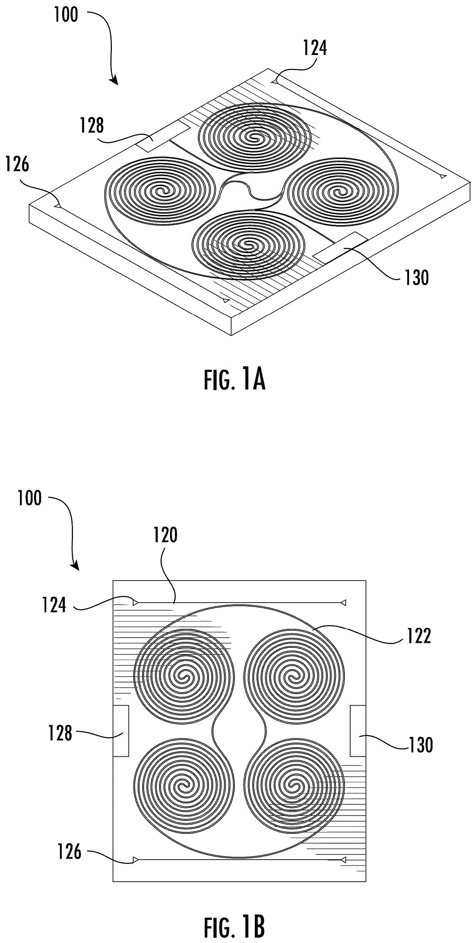

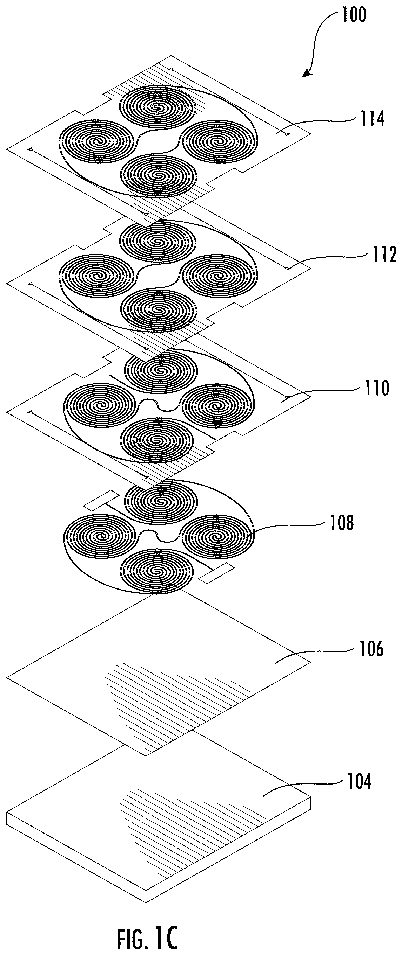

Referring to FIGS. 1A-1C, a photonic gas sensor chip 100 in accordance with at least some embodiments of the present disclosure is illustrated. The photonic gas sensor chip 100 may comprise a layered structure designed for enhanced gas sensing applications using dual frequency comb spectroscopy techniques. The photonic gas sensor chip 100 may be manufactured using semiconductor fabrication processes that enable precise control over layer thickness, material composition, and structural geometry.

The photonic gas sensor chip 100 may include a substrate 104. In some embodiments, the substrate 104 may comprise silicon material. The substrate 104 may alternatively comprise other semiconductor materials or insulating materials. The substrate 104 may serve as the foundational layer upon which other layers and/or components of the photonic gas sensor chip 100 are formed, deposited, or disposed. The substrate 104 may provide mechanical support. In some embodiments, the substrate 104 may have a substantially planar surface that enables precise deposition and patterning of subsequent layers through various techniques such as, but not limited to, photolithographic and etching techniques.

A silicon oxide layer 106 may be disposed on and/or above the substrate 104, as illustrated in FIGS. 1A-1C. The silicon oxide layer 106 may comprise silicon oxide (SiO2) material. In some embodiments, the silicon oxide layer 106 may function as an insulating layer between the substrate 104 and overlying components. In some embodiments, the silicon oxide layer 106 may be formed through thermal oxidation processes or chemical vapor deposition techniques. Thermal oxidation processes may involve exposing the silicon substrate to an oxidizing environment at elevated temperature to form a silicon oxide layer through the chemical reaction of silicon with oxygen or water vapor. Chemical vapor deposition may comprise a thin-film deposition process in which gaseous reactants are introduced into a reaction chamber and undergo chemical reactions at or near a heated substrate surface to form a solid film.

A heating element 108 may be disposed on and/or above the silicon oxide layer 106. The heating element 108 may be configured to provide controlled thermal energy to, for example, adjust optical properties of the overlying nitride waveguide 112 through thermal expansion and thermal refractive index change. In some embodiments, the heating element 108 may be patterned or otherwise constructed from aluminum material. The aluminum material may be selected for its favorable thermal and electrical properties, including high thermal conductivity, low electrical resistance, and compatibility with standard semiconductor fabrication processes. The aluminum material may enable efficient conversion of electrical energy to thermal energy while providing uniform heat distribution across the heating element 108.

A silicon oxide base 110 may be disposed on and/or above the heating element 108. The silicon oxide base 110 may comprise a planarization layer that provides a smooth, uniform surface for subsequent layer deposition. In some examples, the silicon oxide base 110 may be formed through chemical vapor deposition followed by chemical mechanical polishing to achieve the desired surface planarity and thickness uniformity.

A nitride waveguide 112 may be disposed on and/or above the silicon oxide base 110. The nitride waveguide 112 may have a ribbed structure designed to minimize optical losses while maximizing light confinement. In some embodiments, the nitride waveguide 112 may be formed through deposition of a nitride layer (e.g., silicon nitride layer) followed by photolithographic patterning and etching to create the waveguide structure with ribs. The silicon nitride material may exhibit thermal stability characteristics that enable reliable operation across a range of operating temperatures. The silicon nitride material may exhibit high refractive index contrast with the surrounding silicon oxide layers. The ribbed structure of the nitride waveguide 112 may comprise raised portions that extend above a base layer, creating a waveguide structure that enhances light confinement compared to planar waveguide structures. The rib width and height may be optimized to achieve low optical loss. The ribbed structure may provide enhanced light confinement while reducing scattering losses that can occur at waveguide interfaces.

The nitride waveguide 112 may comprise an input waveguide 120, an extended resonant ring waveguide 122, and an output waveguide 127. In some embodiments, each of the input waveguide and output waveguide may comprise a straight bus waveguide. In this regard, in such some embodiments, the input waveguide and output waveguides may have a linear waveguide structure that provides a direct optical path for guiding light between optical components without significant curvature or bending. The input waveguide 120 and the output waveguide 127 may function as an optical transmission medium that carries optical signals (e.g., light) to and from the extended resonant ring waveguide 122. The extended resonant ring waveguide 122 may be positioned between the input waveguide 120 and the output waveguide 127.

The nitride waveguide 112 may further include a light input coupler 124 and a light output coupler 126. The light input coupler 124 may be configured to couple light (e.g., transmit light) into the input waveguide 120. The light input coupler 124 may include focusing elements or features that concentrates incident light from external light sources (e.g., laser sources or the like) into the input waveguide 120 while minimizing coupling losses. Such focusing elements may include grating features, adiabatic tapers, or the like. In this regard, in some embodiments, the light input coupler 124 may be or otherwise comprise an input grating coupler having a series of periodic grating features etched, formed, and/or positioned in or on the silicon nitride waveguide 112 to couple light from external light sources into the input waveguide 120. The periodic features may enable phase matching between incident light and guided modes within the extended resonant ring waveguide structure. In some embodiments, the grating coupler may be optimized for specific wavelengths and incident angles to maximize coupling efficiency while minimizing optical losses.

The periodic grating features may include ridges, grooves, or other surface modulations that create a periodic variation in the effective refractive index of the waveguide. The periodic spacing of the grating features may be designed to achieve phase matching between incident light from external light sources and guided optical modes within the silicon nitride waveguide 112 (e.g., extended resonant ring waveguide structure thereof). The period spacing of the grating features may be selected to optimize coupling efficiency for specific wavelengths used in gas spectroscopy applications, which, for example, may be in the near-infrared wavelength region where many gas molecules exhibit characteristic absorption features. In some embodiments, the light input coupler 124 may be configured to accept light incident on the light input coupler 124 from an external light source, such as a laser source, at a specific angle relative to the surface normal of the photonic gas sensor chip 100. The angular acceptance of the light input coupler 124 (e.g., input grating coupler) may be designed to accommodate practical alignment tolerances while maintaining high coupling efficiency.

The light output coupler 126 may be configured to output light from the extended resonant ring waveguide structure, for example, for detection and/or analysis. Specifically, the light output coupler 126 may be configured to extract light out of the output waveguide 127 of the extended resonant ring waveguide structure. In some embodiments, the light output coupler 126 may be or comprise an output grating coupler comprising a series of periodic grating features etched, formed, or positioned in or on the silicon nitride waveguide 112 to extract light out from the output waveguide 127. The periodic grating features of the output grating coupler may be similar to those of the input grating coupler.

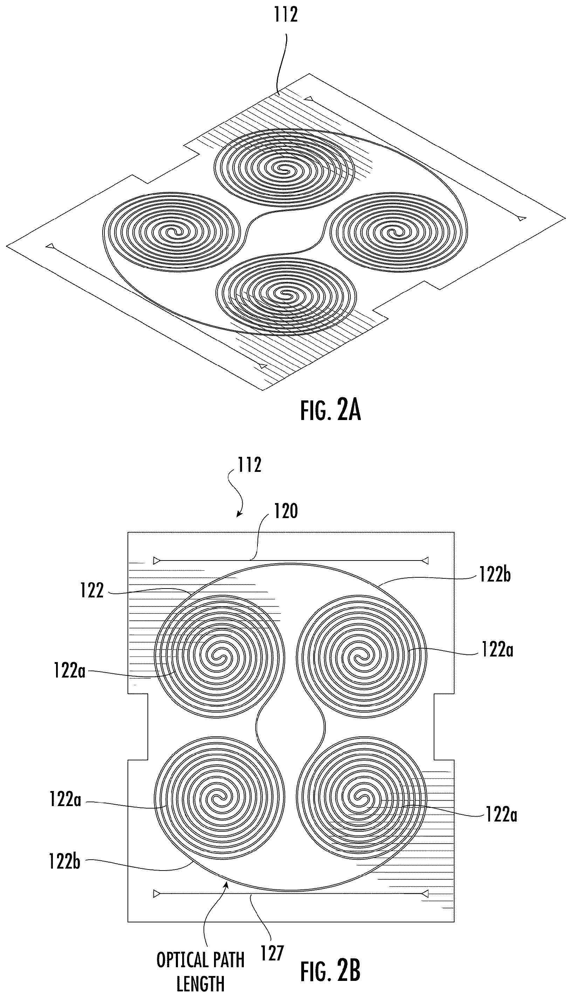

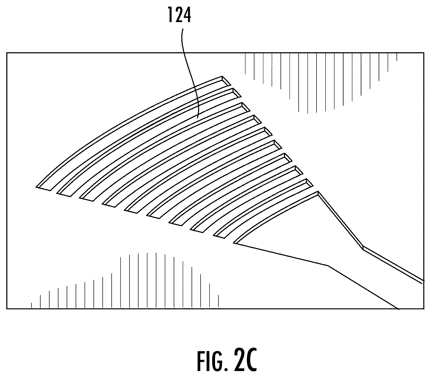

As shown in FIGS. 2A-2C, the extended resonant ring waveguide 122 may comprise a resonant ring structure (also referred to herein interchangeably as resonant ring or ring resonator) with spiral waveguides sections 122a that extends the optical path length of the resonant ring and reduces resonant frequency. As shown in FIGS. 2A-2C, the spiral waveguide sections 122a may be defined, formed, positioned, or otherwise integrated within the central area of the extended resonant ring waveguide 122, maximizing the utilization of available space for gas sensing applications. In some embodiments, the spiral waveguide sections 122a may be arranged at regular angular intervals around a central axis of the extended resonant ring waveguide. The integration of the spiral waveguide sections 122a within the resonator ring may be achieved through careful design of the waveguide routing and connection points to maintain optical continuity and minimize insertion losses. In some embodiments, the spiral waveguide sections 122a may be designed with gradual curvature transitions to reduce optical losses associated with sharp bends or discontinuities in the waveguide structure.

The extended resonant ring waveguide configuration provides enhanced optical interaction with gas samples while maintaining a compact physical footprint suitable for chip-scale applications. The extended resonant ring waveguide configuration enables dual frequency comb spectroscopy (DCS) gas sensing applications by locking the resonant frequency to the dual frequency comb spectroscopy (DCS) repetition frequency and effectively enhancing gas sensor sensitivity.

In the depicted example shown in FIGS. 2A-B, the extended resonant ring waveguide 122 comprises four spiral waveguide sections 122a. In some other embodiments, the extended resonant ring waveguide 122 may comprise more or less than four spiral waveguide sections 122a. The spiral waveguide sections 122a may be symmetrically positioned within the extended resonant ring waveguide 122 and arranged in a symmetric pattern that fills the center space of the resonant ring structure. For example, the four spiral waveguide sections 122a may be arranged at regular angular intervals around a central axis of the extended resonant ring waveguide. The symmetric positioning of the spiral waveguide sections 122a may provide uniform optical path extension while maintaining balanced optical characteristics throughout the extended resonant ring waveguide 122.

The spiral waveguide sections 122a may be formed as continuous waveguide structures (continuous waveguides) that follow curved paths within the interior region of the extended resonant ring waveguide 122. Each spiral waveguide section 122a may comprise multiple turns or loops that increase the total optical path length while remaining within the physical boundaries defined by the resonant ring structure. The spiral geometry may enable the optical path length to be extended beyond what would be achievable with conventional ring configuration of the same diameter.

In some embodiments, the spiral waveguide sections 122a may be positioned at regular angular intervals around the central axis of the extended resonant ring waveguide 122, with each spiral waveguide section 122a occupying approximately 90 degrees of the available angular space. The symmetric arrangement may ensure that optical signals (e.g., light) propagating through the extended resonant ring waveguide 122 undergo uniform path length extensions regardless of their starting position within the extended resonant ring waveguide 122. This symmetry may reduce optical losses and maintain consistent resonance characteristics across the entire ring resonator structure.

In some embodiments, each spiral waveguide section 122a may be designed with gradually varying curvature to minimize optical losses associated with sharp bends or abrupt direction changes. The spiral waveguide sections 122a may transition smoothly from the ring perimeter 122b (e.g., core resonant ring) into the spiral geometry and back to the ring perimeter 122b, maintaining optical continuity throughout the extended path. The smooth transitions may reduce scattering losses and preserve the coherence properties of optical signals propagating through the extended resonant ring waveguide 122.

The spiral waveguide sections 122a may be interconnected through the resonant ring structure to form a continuous optical path that encompasses both the ring perimeter 122b and the spiral waveguide sections 122a. Light propagating through the extended resonant ring waveguide 122 may travel through each spiral waveguide section 122a in sequence, accumulating additional optical path length with each spiral traversal. The interconnected design may ensure that each spiral waveguide section 122a contributes to the total optical path length extension.

The spacing between adjacent turns (e.g., adjacent spiral segments) within each spiral waveguide section 122a may be optimized to prevent optical coupling between neighboring spiral segments while maximizing the number of turns that can be accommodated within the available space. The spiral pitch may be selected to maintain sufficient separation between spiral segments to avoid unwanted optical crosstalk while achieving maximum path length extension. The spiral pitch may comprise the distance between adjacent turns or loops of a spiral waveguide section 122a measured in a direction perpendicular to the spiral path. Optical crosstalk may comprise unwanted optical coupling or interference between adjacent spiral segments or neighboring spiral waveguide sections that can degrade the performance of the extended resonant ring waveguide 122. An optical crosstalk may occur when light propagating in one spiral segment unintentionally couples into an adjacent spiral segment due to insufficient spacing or inadequate optical isolation between the waveguide structures.

In some embodiments, the spiral waveguide sections 122a may be designed to maintain the same cross-sectional geometry as the core ring, ensuring consistent optical mode characteristics throughout the extended optical path. The ribbed silicon nitride structure may be preserved throughout the spiral waveguide sections 122a, providing uniform light confinement and minimizing modal dispersion effects that could degrade spectroscopic performance.

By way of example, the extended resonant ring waveguide 122 may achieve an effective optical path length of approximately 150mm while maintaining a ring diameter of approximately 3mm. The optical path length may comprise the total distance that light travels through the extended resonant ring waveguide 122, accounting for both the physical path distance and the refractive index of the medium through which the light propagates. For example, the optical path length may encompass the cumulative distance light travels through the ring resonator structure, including the ring perimeter 122b and the spiral waveguide sections 122a.

The extended optical path length enables enhanced interaction between the propagating light and gas molecules in the evanescent field, thereby increasing the sensitivity of gas detection operations. For example, the extended optical path length created by the spiral extended resonant ring configuration may enable evanescent field detection for gas sensing applications. Such gas sensing applications may be performed by evanescent field detection of the extended resonant ring waveguide 122, where sample gas over the photonic gas sensor chip 100

(e.g., over the extended resonant ring waveguide 122 thereof) may create amplitude profile changes of the input light beam (e.g., laser comb signal from a dual comb source). The evanescent field may extend beyond the physical boundaries of the waveguide, allowing interaction with gas molecules in the surrounding environment.

Evanescent field detection may utilize evanescent electromagnetic field to detect and analyze gas molecules present in the surrounding medium. The evanescent field may comprise electromagnetic energy that propagates along the waveguide and which may extend into the medium surrounding the waveguide. Evanescent field detection may enable gas molecules to interact with the electromagnetic field extending from the silicon nitride waveguide 112, causing measurable changes in the optical properties of the propagating light, such as amplitude, phase, or polarization modifications, which can be detected and analyzed to determine the presence, concentration, or identity of target gas species. The extended optical path length provided by the spiral waveguide sections 122a enhances the evanescent field detection capability by increasing the total interaction volume and time between the evanescent field and gas molecules, thereby improving the sensitivity of gas detection operations compared to conventional single-pass optical sensing systems.

In some embodiments, the spiral extended resonant ring configuration may provide an effective extended optical path length of approximately 150 millimeters (150mm) within a ring resonator having a diameter of approximately 3 millimeters, as described above, resulting in a resonant enhanced equivalent detection length that may exceed 100 meters for low concentration gas sensing applications. The extended optical path length combined with resonant enhancement effects may amplify the interaction between light and gas molecules, increasing the sensitivity of gas detection compared to conventional single-pass optical systems. In some examples, the resonant enhanced equivalent detection length may be over 1000 times greater than conventional systems, enabling detection of trace gas concentrations that may be difficult to measure using traditional optical sensing techniques.

The compact ring diameter may facilitate integration into miniaturized sensor packages, while the extended optical path length may enable resonance at approximately 2GHz to match dual frequency comb spectroscopy repetition frequencies. The combination of compact physical dimensions and extended optical path length addresses the technical challenges associated with frequency matching in conventional ring resonator designs. The relationship between optical path length and resonant frequency may be governed by the fundamental resonance condition, where the optical path length equals an integer multiple of the wavelength of the circulating light. In conventional ring resonators, the optical path length may be determined primarily by the circumference of the ring structure. The incorporation of the spiral waveguide sections 122a within the extended resonant ring waveguide 122 may increase the effective optical path length by approximately 8 times compared to a conventional ring of the same diameter.

The 2GHz resonant frequency achieved through the extended optical path configuration may correspond to a free spectral range that enables precise matching with typical dual frequency comb spectroscopy systems. Dual frequency comb spectroscopy systems may generally operate with repetition frequencies in the range of 100kHz to 1GHz, with 200kHz representing an optimal frequency for many gas sensing applications. The ability to achieve this resonant frequency of 2GHz (matching 10 multiples of 200kHz comb repetition frequency) within a compact 3mm diameter footprint may represent a significant advancement over conventional approaches that would require ring diameters of 24mm or larger to achieve equivalent resonant frequencies.

The extended effective optical path length of approximately 150mm may provide enhanced sensitivity for gas detection through increased interaction between the evanescent field and gas molecules present in the sensing environment. The evanescent field penetration depth may extend approximately 100-500 nanometers beyond the waveguide surface, depending on the wavelength and refractive index contrast between the silicon nitride waveguide and the surrounding medium. The 150mm effective optical path length may provide substantially more opportunities for gas molecules to interact with the evanescent field compared to conventional ring resonators with shorter optical path lengths.

The compact 3mm ring diameter may enable integration into can packages such as TO39 can packages, or similar size sensor housings while maintaining compatibility with standard semiconductor fabrication processes. The 3mm diameter may represent an optimal balance between manufacturing feasibility and optical performance, as smaller diameters may introduce excessive bending losses in the curved segments of the spiral waveguide sections 122a, while larger diameters may compromise the compact form factor advantages.

The frequency matching capability enabled by the extended optical path configuration (e.g., spiral extended resonant ring configuration) may eliminate the need for complex external frequency conversion systems that would otherwise be required to interface conventional ring resonators with dual frequency comb spectroscopy sources (also referred to herein interchangeably as dual comb source). The direct frequency matching may reduce system complexity, improve measurement stability, and enhance overall gas sensor performance by maintaining optimal resonance conditions throughout the gas sensing operation.

The technical challenges addressed by the enhanced ring resonator design (e.g., spiral extended resonant ring configuration) may include the fundamental trade-off between resonator size and resonant frequency that limits conventional approaches. In traditional ring resonator designs, achieving lower resonant frequencies may require larger ring diameters, which may create manufacturing challenges and limit the practical applications of the ring resonator technology. For example, ring resonators with diameters exceeding 10mm may become difficult to fabricate using standard semiconductor processing techniques and may be incompatible with compact sensor packaging requirements for portable or embedded gas sensing applications.

The spiral extended ring configuration may overcome these limitations by decoupling the relationship between physical ring diameter and optical path length. The spiral waveguide sections 122a may effectively multiply the optical path length by a factor of, for example, approximately 8 compared to a conventional ring of the same diameter, enabling the achievement of low resonant frequencies within compact physical dimensions. This approach may preserve the manufacturing advantages of small-scale semiconductor fabrication while achieving the optical performance characteristics of much larger resonator structures.

A cover 114 may be disposed on the nitride waveguide 112. The cover 114 may comprise a conformal silicon oxide layer that contributes to reduction of optical losses in the nitride waveguide 112. In some embodiments, the cover 114 (e.g., conformal silicon oxide layer) may be formed through chemical vapor deposition processes, atomic layer deposition processes, or other processes that enable precise control over layer thickness and conformality to the underlying nitride waveguide 112. The conformal nature of the silicon oxide layer may ensure uniform coverage over the ribbed structure of the silicon nitride waveguide 112, providing consistent optical properties across the waveguide geometry. In some embodiments, the thickness of the cover 114 (e.g., conformal silicon oxide layer) may be selected to optimize optical confinement while maintaining compatibility with evanescent field sensing requirements.

The cover 114 (e.g., conformal silicon oxide layer) may serve as a protective layer while also contributing to optical confinement within the nitride waveguide 112. The protective function of the cover 114 (e.g., conformal silicon oxide layer) may shield the silicon nitride waveguide 112 from environmental contaminants and physical damage during handling and operation. The silicon oxide material may provide chemical resistance to various gases and environmental conditions encountered in gas sensing applications, ensuring long-term stability of the optical properties. In some embodiments, the cover 114 (e.g., conformal silicon oxide layer) may be designed with specific surface treatments or coatings that enhance the interaction between the evanescent field and target gas molecules while maintaining protection for the underlying waveguide.

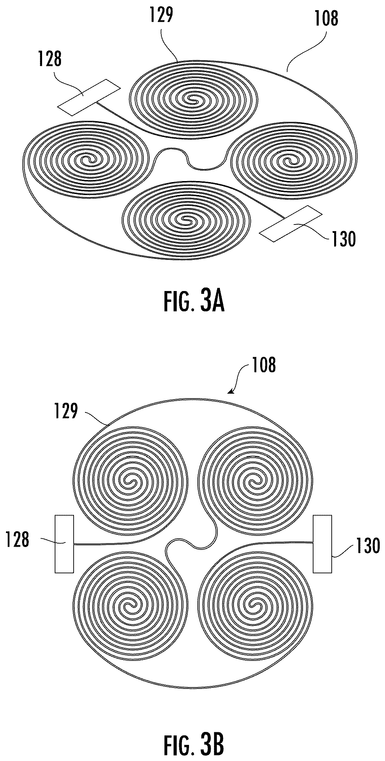

Referring to FIGS. 3A and 3B, the heating element 108 may be configured in a spiral pattern that extends across a planar surface. The heating element 108 may include a heating current input pad 128, a spiral heating coil 129 defining spiral loops (e.g., multiple heating coils), and a heating current output pad 130. The heating current input pad 128 and heating current output pad 130 may be coupled to the spiral heating coil 129. As shown in FIG. 3A, the spiral heating coil 129 may have a shape that corresponds to the shape of the extended resonant ring waveguide 122. For example, the spiral loops (e.g., heating coils) defined by the spiral heating coil 129 may correspond to the spiral waveguide sections 122a of the extended resonant ring waveguide 122.

The heating current input pad 128 may provide an electrical connection point for supplying current to the heating element 108, while the heating current output pad 130 may provide an electrical connection point for current to exit the heating element 108, completing the electrical circuit for thermal control operations. The heating current input pad 128 may be configured to receive electrical current from an external power source or control circuit. The heating current output pad 130 may work in conjunction with the heating current input pad 128 to establish a complete current path through the heating element structure. The heating current output pad 130 may be positioned opposite to or at a different location from the heating current input pad 128 to enable proper current flow distribution through the heating element structure.

The heating current input pad 128 and heating current output pad 130 may be electrically connected to the aluminum heating element through conductive traces formed in the same layer (e.g., metal layer) as the spiral heating coil 129. The electrical connections may provide low-resistance pathways for current flow, ensuring efficient thermal generation and precise temperature control. In some embodiments, the electrical connections may be designed to minimize thermal losses while providing reliable electrical connectivity for sustained heating operations.

The spiral heating coil 129 may include multiple concentric loops that provide uniform heat distribution across the nitride waveguide 112 or at least across the extended resonant ring waveguide 122. The spiral configuration of the heating element 108 may allow for controlled heating across the silicon nitride waveguide 112 or extended resonant ring waveguide 122 thereof while maintaining uniform temperature distribution. The spiral pattern may maximize the heating area coverage while providing efficient electrical resistance characteristics for thermal generation.

The heating element 108 may be positioned below the nitride waveguide 112 to fine tune the ring resonant frequency. The heating current input pad 128 may be positioned on one side of the heating element 108, and the heating current output pad 130 may be positioned on the opposite side, providing electrical access points for current flow through the spiral heating structure. The spiral configuration may fill the area beneath the extended resonant ring waveguide, providing controlled heating across the entire resonator structure. The heating element 108 may be superimposed with the nitride waveguide 112. The example, the spiral loops (e.g., heating coils) of the heating element 108 may arranged below the extended resonant ring waveguide.

The heating element 108 may be configured to introduce thermal expansion and thermal refractive index change that adjusts a length and refractive index of the silicon nitride waveguide 112, enabling precise control over the optical path length and resonant characteristics of the silicon nitride waveguide 112. The heating element 108 may be configured to lock (e.g., match or similar terms) a resonant frequency of the extended ring resonator to a DCS repetition frequency through precise thermal control. The thermal expansion and thermal refractive index change induced by the heating element 108 may adjust the effective length of the silicon nitride waveguide 112, thereby modifying the optical path length and shifting the resonant frequency of the extended ring resonator. In some embodiments, the thermal tuning capability may enable fine adjustment of the resonant frequency to achieve precise matching with DCS repetition frequencies.

The heating element 108 may provide rapid thermal response characteristics that enable dynamic frequency tuning during gas sensing operations. The thermal expansion and thermal refractive index change of the silicon nitride waveguide material may create changes in the effective optical path length, allowing for real-time adjustment of the resonant frequency to maintain optimal resonance conditions. In some embodiments, the thermal tuning range may be sufficient to compensate for manufacturing variations and environmental factors that could affect the resonant frequency of the extended ring resonator.

The aluminum heating element 108 may enable formation of a resonant enhanced equivalent detection length that may be over 1000 times greater than conventional optical sensing systems. For example, the heating element 108 may be configured to introduce thermal expansion and thermal refractive index change that adjusts a length and refractive index of the silicon nitride waveguide 112 to lock a resonant frequency of the nitride waveguide 112 to a DCS repetition frequency to form resonant enhanced equivalent detection length over 1000 times. The thermal tuning capability may maintain optimal resonance conditions that maximize the interaction between light and gas molecules, enhancing the sensitivity of gas detection for low concentration applications. The precise frequency matching achieved through thermal control may enable stable operation of the enhanced ring resonator in dual frequency comb spectroscopy systems.

The aluminum heating element 108 may be fabricated with specific geometric parameters that optimize thermal efficiency while maintaining compatibility with semiconductor manufacturing processes. In some examples, the spiral pattern may comprise a continuous conductive trace. The aluminum material selection for the heating element 108 may provide several advantages over alternative heating materials, including high thermal conductivity, low electrical resistivity, and compatibility with CMOS fabrication processes. In some embodiments, the thermal expansion mechanism may operate through direct thermal expansion of the silicon nitride material and thermo-optic effects that modify the refractive index of the waveguide material. The combined effects of thermal expansion and thermo-optic changes may provide a total optical path length change that enables precise frequency tuning.

In some embodiments, the electrical resistance of the aluminum heating element 108 may be designed to provide optimal power dissipation characteristics for the required thermal tuning range. The resistance value may be selected to enable operation at practical voltage levels ranging while providing sufficient thermal power to achieve the desired frequency tuning range. In some embodiments, the electrical connections between the heating current input pad 128 and heating current output pad 130 may be designed to minimize parasitic inductance and capacitance that could affect the frequency response of the thermal control system. The trace routing may be optimized to minimize electromagnetic interference with sensitive optical detection circuits and to provide adequate separation from other electrical components on the photonic gas sensor chip 100. In some embodiments, the thermal isolation characteristics of the silicon oxide base 110 and silicon oxide layer 106 may be optimized to concentrate thermal energy within the ring resonator region while minimizing heat loss to the silicon substrate 104.

The frequency tuning range achievable through thermal control may be sufficient to compensate for manufacturing variations in ring resonator dimensions and to maintain optimal resonance conditions across different operating temperatures. In some embodiments, the frequency tuning range may extend from approximately 1 GHz to approximately 10 GHz, depending on the temperature range of the aluminum heating element 108 and the thermal expansion and thermal refractive index change characteristics of the silicon nitride material. The tuning range may enable the ring resonator to maintain resonance lock with dual frequency comb spectroscopy sources across temperature variations, ensuring stable operation in diverse environmental conditions.

### Example Photonic Gas Sensor

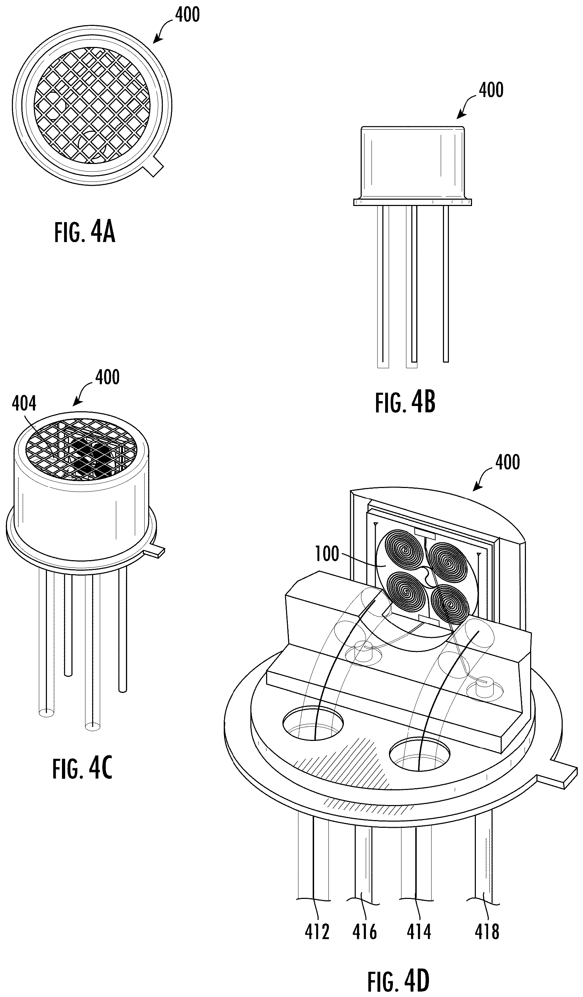

Referring to FIGS. 4A-4D, a photonic gas sensor 400 may incorporate the photonic gas sensor chip 100 within an assembly configuration designed for gas sensing applications. The photonic gas sensor 400 may provide a packaged solution that enables deployment in various industrial, environmental, and healthcare monitoring systems while maintaining the enhanced sensitivity characteristics of the underlying photonic gas sensor chip 100.

As shown in FIG. 4A, the photonic gas sensor 400 may include a gas window 404 positioned on an upper surface of the assembly. The gas window 404 may allow gas samples to contact the photonic gas sensor chip 100 while providing structural protection for the underlying waveguide components. In some embodiments, the gas window 404 may comprise a porous material that enables gas flow while preventing contamination or physical damage to the optical components within the photonic gas sensor 400.

The porous material of the gas window 404 may be selected to provide optimal gas permeability while maintaining structural integrity and protection for the photonic gas sensor chip 100. In some embodiments, the gas window 404 may comprise a mesh structure with precisely controlled pore sizes that allow target gas molecules to pass through while blocking larger particles or contaminants that could interfere with optical sensing operations. The mesh structure may be formed from materials such as stainless steel, ceramic, or polymer materials that provide chemical resistance to various gas environments encountered in industrial, environmental, or healthcare applications. In this regard, in some embodiments, the gas window 404 may be embodied as a gas mesh window.

The pore size distribution of the gas window 404 may be optimized to enable rapid gas exchange between the external environment and the evanescent field region of the silicon nitride waveguide 112 while preventing the ingress of dust, moisture, or other environmental contaminants. The pore density may be selected to maximize the open area available for gas flow while maintaining sufficient structural strength to protect the underlying photonic components.

The gas window 404 may be designed to minimize optical interference with the evanescent field sensing mechanism while providing unrestricted access for gas molecules to interact with the nitride waveguide 112. The thickness of the gas window 404 may be optimized to provide adequate mechanical protection without creating significant diffusion barriers that could slow the response time of gas sensing operations.

The material selection for the gas window 404 may consider chemical compatibility with target gases to prevent unwanted chemical reactions or adsorption effects that could affect measurement accuracy. The gas window material may exhibit low adsorption characteristics for target gas molecules to ensure rapid equilibration between external gas concentrations and the gas concentration in the evanescent field region. In some embodiments, the gas window 404 may include surface treatments or coatings that enhance chemical resistance while maintaining optimal gas permeability characteristics.

The mounting configuration of the gas window 404 may provide a hermetic seal around the perimeter while allowing controlled gas access through the porous central region. The hermetic seal may prevent uncontrolled gas leakage that could affect the accuracy of gas concentration measurements or compromise the protection of internal components. In some embodiments, the gas window 404 may be secured to the photonic gas sensor 400 using adhesive bonding, mechanical fastening, or welding techniques that provide reliable long-term sealing performance.

The gas window 404 may be positioned at an optimal distance from the silicon nitride waveguide 112 to enable efficient gas transport to the evanescent field region while providing adequate clearance for thermal expansion and mechanical tolerances. The spacing between the gas window 404 and the waveguide surface may be designed to minimize gas diffusion time while preventing mechanical contact that could damage the sensitive optical components. In some embodiments, the spacing may range from approximately 100 micrometers to approximately 1 millimeter, depending on the specific sensor design and packaging requirements.

With continued reference to FIGS. 4A-4D, the photonic gas sensor 400 may include a light inlet 412 configured for receiving an input light beam from external optical sources, such as dual comb sources. The light inlet 412 may be positioned to align with the light input coupler 124 of the photonic gas sensor chip 100, enabling efficient coupling of optical signals into the silicon nitride waveguide 112. In some embodiments, the light inlet 412 may be configured to accommodate fiber optic connections that provide optical coupling between external dual frequency comb spectroscopy sources and the ring resonator structure.

The light inlet 412 may comprise an optical interface structure designed to optimize coupling efficiency between external optical sources and the photonic gas sensor chip 100. The optical interface structure may include precision alignment features that ensure accurate positioning of incident light beams relative to the light input coupler 124, minimizing coupling losses and maintaining optimal optical performance. In some embodiments, the light inlet 412 may include mechanical alignment guides or reference surfaces that facilitate repeatable positioning of fiber optic connectors or free-space optical components during assembly and operation.

The fiber optic connections accommodated by the light inlet 412 may comprise single-mode optical fibers that preserve the coherence characteristics of dual frequency comb spectroscopy sources. Single-mode fibers may maintain the spatial and temporal coherence properties of the optical signals, which may be essential for achieving optimal performance in dual frequency comb spectroscopy applications. The fiber optic connections may utilize standard connector types such as FC/PC, SC/PC, or LC connectors that provide reliable optical coupling while enabling convenient connection and disconnection for system maintenance or reconfiguration.

In some embodiments, the light inlet 412 may include anti-reflection coatings or optical matching elements that minimize reflection losses at the interface between the fiber optic connection and the photonic gas sensor chip 100. Anti-reflection coatings may be applied to optical surfaces within the light inlet 412 to reduce Fresnel reflections that could degrade coupling efficiency or create unwanted optical feedback to the external light source. The optical matching elements may comprise index-matching materials or gradient-index optical elements that provide smooth refractive index transitions between different optical media.

The positioning of the light inlet 412 may be optimized to provide direct optical access to the light input coupler 124 while maintaining mechanical protection for the sensitive photonic components. The light inlet 412 may be recessed within the housing structure to prevent accidental contact with the photonic gas sensor chip 100 while providing adequate clearance for fiber optic connectors or other optical coupling devices. In some embodiments, the light inlet 412 may include protective covers or shutters that shield the optical interface when not in use, preventing contamination or damage to the optical surfaces.

The light inlet 412 may be designed to accommodate various types of external optical sources beyond dual frequency comb spectroscopy systems, including tunable laser sources, broadband light sources, or other coherent optical sources suitable for gas sensing applications. The versatility of the optical interface may enable the photonic gas sensor 400 to be integrated into different types of spectroscopic systems depending on the specific application requirements and performance objectives. The light inlet 412 may include adjustable coupling optics that enable optimization of the coupling efficiency for different types of external light sources.

In some embodiments, the light inlet 412 may incorporate optical isolation elements that prevent unwanted optical feedback from the photonic gas sensor chip 100 to the external light source. Optical isolators may comprise Faraday rotators or other magneto-optic devices that allow light to pass in one direction while blocking reflected light from traveling back to the source. The optical isolation may protect sensitive external light sources from destabilization caused by optical feedback, ensuring stable operation of the dual frequency comb spectroscopy system.

The mechanical design of the light inlet 412 may include strain relief features that protect fiber optic connections from mechanical stress during installation and operation. Strain relief elements may comprise flexible boots, bend radius limiters, or mechanical clamps that prevent excessive bending or tension in the optical fibers. The strain relief features may ensure long-term reliability of the optical connections while maintaining optimal coupling performance throughout the operational lifetime of the photonic gas sensor 400.

The light inlet 412 may be hermetically sealed to prevent gas leakage while maintaining optical access to the photonic gas sensor chip 100. Hermetic sealing may be achieved through the use of optical feedthroughs that provide gas-tight seals around fiber optic connections or optical windows. The hermetic sealing may ensure that gas samples are contained within the designated sensing chamber while preventing contamination of the optical components or interference with gas concentration measurements.

A light outlet 414 may be provided through which optical signals exit the photonic gas sensor 400 after interaction with gas samples within the assembly, as illustrated in FIGS. 4A-4D. The light outlet 414 may be positioned to align with the light output coupler 126 of the photonic gas sensor chip 100, enabling efficient extraction of optical signals for subsequent detection and analysis. The light outlet 414 may accommodate fiber optic connections that transport optical signals to external photodetectors and signal processing systems.

The light outlet 414 may comprise an optical interface structure designed to optimize extraction efficiency of optical signals from the photonic gas sensor chip 100 while maintaining signal integrity for subsequent analysis operations. The optical interface structure may include precision alignment features that ensure accurate positioning of the light output coupler 126 relative to external optical collection systems, minimizing extraction losses and preserving the spectroscopic information contained within the optical signals. In some embodiments, the light outlet 414 may include mechanical alignment guides or reference surfaces that facilitate repeatable positioning of fiber optic connectors or free-space optical collection devices during assembly and operation.

The fiber optic connections accommodated by the light outlet 414 may comprise single-mode optical fibers that preserve the coherence and spectroscopic characteristics of optical signals that have interacted with gas samples within the chamber 424. Single-mode fibers may maintain the spatial and temporal properties of the optical signals, which may be essential for preserving the spectroscopic information required for accurate gas concentration measurements in dual frequency comb spectroscopy applications. The fiber optic connections may utilize standard connector types such as FC/PC, SC/PC, or LC connectors that provide reliable optical coupling while enabling convenient connection and disconnection for system maintenance or reconfiguration.

In some embodiments, the light outlet 414 may include anti-reflection coatings or optical matching elements that minimize reflection losses at the interface between the photonic gas sensor chip 100 and the external optical collection system. Anti-reflection coatings may be applied to optical surfaces within the light outlet 414 to reduce Fresnel reflections that could degrade extraction efficiency or create unwanted optical interference that might affect measurement accuracy. The optical matching elements may comprise index-matching materials or gradient-index optical elements that provide smooth refractive index transitions between the silicon nitride waveguide 112 and the external optical medium.

The positioning of the light outlet 414 may be optimized to provide direct optical access to the light output coupler 126 while maintaining mechanical protection for the sensitive photonic components. The light outlet 414 may be recessed within the housing structure to prevent accidental contact with the photonic gas sensor chip 100 while providing adequate clearance for fiber optic connectors or other optical collection devices. In some embodiments, the light outlet 414 may include protective covers or shutters that shield the optical interface when not in use, preventing contamination or damage to the optical surfaces.

The light outlet 414 may be designed to accommodate various types of external photodetectors and signal processing systems, including photodiodes, photomultiplier tubes, or specialized detectors optimized for dual frequency comb spectroscopy applications. The versatility of the optical interface may enable the photonic gas sensor 400 to be integrated into different types of spectroscopic analysis systems depending on the specific application requirements and performance objectives. The light outlet 414 may include adjustable collection optics that enable optimization of the extraction efficiency for different types of external detection systems.

In some embodiments, the light outlet 414 may incorporate optical filtering elements that selectively transmit specific wavelength ranges while blocking unwanted optical signals that could interfere with gas sensing measurements. Optical filters may comprise interference filters, absorption filters, or other wavelength-selective elements that enhance the signal-to-noise ratio of the extracted optical signals. The optical filtering may improve measurement accuracy by reducing background noise and eliminating optical signals that do not contribute to gas sensing information.

The mechanical design of the light outlet 414 may include strain relief features that protect fiber optic connections from mechanical stress during installation and operation. Strain relief elements may comprise flexible boots, bend radius limiters, or mechanical clamps that prevent excessive bending or tension in the optical fibers. The strain relief features may ensure long-term reliability of the optical connections while maintaining optimal extraction performance throughout the operational lifetime of the photonic gas sensor 400.

The light outlet 414 may be hermetically sealed to prevent gas leakage while maintaining optical access from the photonic gas sensor chip 100. Hermetic sealing may be achieved through the use of optical feedthroughs that provide gas-tight seals around fiber optic connections or optical windows. The hermetic sealing may ensure that gas samples are contained within the designated sensing chamber while preventing contamination of the optical components or interference with gas concentration measurements.

The optical signals extracted through the light outlet 414 may contain spectroscopic information that reflects the interaction between the evanescent field of the silicon nitride waveguide 112 and gas molecules present in the chamber 424. The spectroscopic information may include amplitude modulations, phase shifts, or frequency changes that correspond to the absorption characteristics of target gas molecules. The preservation of this spectroscopic information through the light outlet 414 may be critical for achieving the enhanced sensitivity and accuracy capabilities of the dual frequency comb spectroscopy gas sensing system.

As further shown in FIGS. 4A-4D, the photonic gas sensor 400 may include a heater inlet 416 through which electrical connections for thermal control enter the assembly. The heater inlet 416 may provide electrical access to the heating current input pad 128 of the photonic gas sensor chip 100, enabling external control of the aluminum heating element for frequency tuning operations. In some embodiments, the heater inlet 416 may accommodate electrical leads that connect to external control circuits for thermal management.

The heater inlet 416 may comprise a hermetically sealed electrical feedthrough that maintains gas containment within the chamber 424 while providing reliable electrical connectivity to the heating current input pad 128. The hermetic sealing may be achieved through the use of glass-to-metal seals, ceramic feedthroughs, or other sealing technologies that prevent gas leakage while maintaining low electrical resistance for efficient thermal control operations. The electrical feedthrough design may accommodate multiple conductor configurations, including single-wire, twisted-pair, or multi-conductor arrangements depending on the specific thermal control requirements and system integration needs.

The electrical leads accommodated by the heater inlet 416 may comprise insulated conductors with temperature ratings suitable for the thermal environment within the photonic gas sensor 400. The conductor materials may include copper, gold-plated copper, or other conductive materials that provide low electrical resistance and reliable current-carrying capacity for sustained heating operations.

The heater inlet 416 may include strain relief features that protect the electrical connections from mechanical stress during installation, operation, and maintenance activities. Strain relief elements may comprise flexible boots, cable clamps, or mechanical supports that prevent excessive bending or tension in the electrical leads while maintaining reliable electrical connectivity. The strain relief features may be particularly important in applications where the photonic gas sensor 400 may be subject to vibration, thermal cycling, or other environmental stresses that could affect the integrity of electrical connections.

In some embodiments, the heater inlet 416 may incorporate electrical isolation features that prevent unwanted electrical coupling between the thermal control circuits and other electrical systems within the photonic gas sensing system 410. Electrical isolation may be achieved through the use of isolation transformers, optocouplers, or other isolation techniques that maintain electrical safety while enabling precise thermal control. The electrical isolation may protect sensitive control circuits from electrical noise or interference that could affect the accuracy of frequency tuning operations.

The external control circuits connected through the heater inlet 416 may include current regulation circuits that maintain stable current flow through the aluminum heating element regardless of variations in supply voltage or ambient temperature conditions. Current regulation may be achieved through the use of constant current sources, feedback control circuits, or other current control techniques that ensure consistent thermal output from the heating element. The current regulation may enable precise control of the thermal expansion and thermal refractive index change and corresponding frequency tuning of the ring resonator structure.

The heater inlet 416 may be designed to accommodate various types of external control interfaces, including analog control signals, digital control signals, or communication protocols that enable integration with different types of control systems. Analog control interfaces may utilize voltage or current signals that directly control the heating element power, while digital control interfaces may utilize pulse-width modulation, serial communication, or other digital control techniques. The flexibility of the control interface may enable the photonic gas sensor 400 to be integrated into different types of gas monitoring systems depending on the specific application requirements and system architecture.

In some embodiments, the heater inlet 416 may include built-in protection features such as overcurrent protection, overvoltage protection, or thermal protection that prevent damage to the aluminum heating element or other components within the photonic gas sensor chip 100. Protection circuits may include fuses, circuit breakers, or electronic protection devices that automatically disconnect power in the event of fault conditions. The protection features may ensure safe operation of the thermal control system while preventing damage that could affect the long-term reliability of the gas sensing device.

The positioning of the heater inlet 416 may be optimized to provide convenient access for electrical connections while minimizing interference with optical components and gas flow paths within the photonic gas sensor 400. The heater inlet 416 may be located on a side surface or bottom surface of the housing to provide easy access during assembly and maintenance operations while maintaining the compact form factor of the sensor package. The positioning may also consider electromagnetic compatibility requirements to minimize electrical interference with sensitive optical detection circuits.

The electrical connections provided through the heater inlet 416 may enable real-time monitoring of heating element performance, including resistance measurements, temperature feedback, or power consumption monitoring that support advanced thermal control algorithms. Monitoring capabilities may enable closed-loop control of the heating element that automatically adjusts power levels to maintain optimal frequency tuning conditions. The monitoring data may also support predictive maintenance algorithms that can detect degradation in heating element performance before it affects gas sensing accuracy.

A heater outlet 418 may be provided through which electrical connections for thermal control exit the photonic gas sensor 400, as shown in the assembly views of FIGS. 4A-4D. The heater outlet 418 may provide electrical access to the heating current output pad 130 of the photonic gas sensor chip 100, completing the electrical circuit for thermal control of the aluminum heating element. The heater inlet 416 and the heater outlet 418 may enable external control systems to regulate the thermal tuning of the ring resonator frequency.

The heater outlet 418 may comprise a hermetically sealed electrical feedthrough that maintains gas containment within the chamber 424 while providing reliable electrical connectivity to the heating current output pad 130. The hermetic sealing may be achieved through the use of glass-to-metal seals, ceramic feedthroughs, or other sealing technologies that prevent gas leakage while maintaining low electrical resistance for efficient thermal control operations. The electrical feedthrough design may accommodate multiple conductor configurations, including single-wire, twisted-pair, or multi-conductor arrangements depending on the specific thermal control requirements and system integration needs.

The electrical leads accommodated by the heater outlet 418 may comprise insulated conductors with temperature ratings suitable for the thermal environment within the photonic gas sensor 400. The conductor materials may include copper, gold-plated copper, or other conductive materials that provide low electrical resistance and reliable current-carrying capacity for sustained heating operations.

The heater outlet 418 may include strain relief features that protect the electrical connections from mechanical stress during installation, operation, and maintenance activities. Strain relief elements may comprise flexible boots, cable clamps, or mechanical supports that prevent excessive bending or tension in the electrical leads while maintaining reliable electrical connectivity. The strain relief features may be particularly important in applications where the photonic gas sensor 400 may be subject to vibration, thermal cycling, or other environmental stresses that could affect the integrity of electrical connections.

In some embodiments, the heater outlet 418 may incorporate electrical isolation features that prevent unwanted electrical coupling between the thermal control circuits and other electrical systems within the photonic gas sensing system 410. Electrical isolation may be achieved through the use of isolation transformers, optocouplers, or other isolation techniques that maintain electrical safety while enabling precise thermal control. The electrical isolation may protect sensitive control circuits from electrical noise or interference that could affect the accuracy of frequency tuning operations.

The external control circuits connected through the heater outlet 418 may include current regulation circuits that maintain stable current flow through the aluminum heating element regardless of variations in supply voltage or ambient temperature conditions. Current regulation may be achieved through the use of constant current sources, feedback control circuits, or other current control techniques that ensure consistent thermal output from the heating element. The current regulation may enable precise control of the thermal expansion and thermal refractive index change and corresponding frequency tuning of the ring resonator structure.

The heater outlet 418 may be designed to accommodate various types of external control interfaces, including analog control signals, digital control signals, or communication protocols that enable integration with different types of control systems. Analog control interfaces may utilize voltage or current signals that directly control the heating element power, while digital control interfaces may utilize pulse-width modulation, serial communication, or other digital control techniques. The flexibility of the control interface may enable the photonic gas sensor 400 to be integrated into different types of gas monitoring systems depending on the specific application requirements and system architecture.

In some embodiments, the heater outlet 418 may include built-in protection features such as overcurrent protection, overvoltage protection, or thermal protection that prevent damage to the aluminum heating element or other components within the photonic gas sensor chip 100. Protection circuits may include fuses, circuit breakers, or electronic protection devices that automatically disconnect power in the event of fault conditions. The protection features may ensure safe operation of the thermal control system while preventing damage that could affect the long-term reliability of the gas sensing device.

The positioning of the heater outlet 418 may be optimized to provide convenient access for electrical connections while minimizing interference with optical components and gas flow paths within the photonic gas sensor 400. The heater outlet 418 may be located on a side surface or bottom surface of the housing to provide easy access during assembly and maintenance operations while maintaining the compact form factor of the sensor package. The positioning may also consider electromagnetic compatibility requirements to minimize electrical interference with sensitive optical detection circuits.

The electrical connections provided through the heater outlet 418 may enable real-time monitoring of heating element performance, including resistance measurements, temperature feedback, or power consumption monitoring that support advanced thermal control algorithms. Monitoring capabilities may enable closed-loop control of the heating element that automatically adjusts power levels to maintain optimal frequency tuning conditions. The monitoring data may also support predictive maintenance algorithms that can detect degradation in heating element performance before it affects gas sensing accuracy.

The electrical circuit completed by the heater inlet 416 and heater outlet 418 may be designed to operate within specific voltage and current ranges that optimize thermal control performance while ensuring safe operation of the aluminum heating element. In some embodiments, the thermal control circuit may be configured to operate at current values ranging from approximately 10 milliamps to approximately 100 milliamps, depending on the specific thermal tuning requirements and power consumption constraints of the gas sensing application.

Referring to the perspective view of FIG. 4C, the photonic gas sensor 400 may have a cylindrical housing configuration with leads extending from a bottom surface for electrical connections and optical fiber connections. The cylindrical housing may provide a compact form factor suitable for integration into various gas monitoring systems while protecting the internal components from environmental factors. In some embodiments, the housing may comprise a TO39 can package configuration that provides standardized dimensions and connection interfaces for industrial applications.

### Example Photonic Gas Sensing System

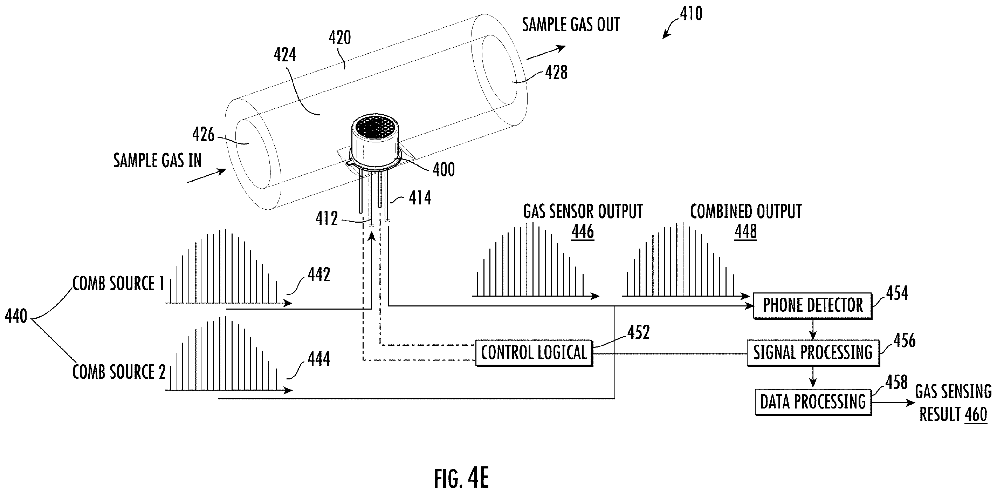

Referring to FIG. 4E, a photonic gas sensing system 410 may incorporate the photonic gas sensor 400 within a system configuration designed for dual frequency comb spectroscopy gas sensing applications. The photonic gas sensing system 410 may provide a gas sensing system that combines optical, thermal, and signal processing components to enable precise gas detection and analysis with enhanced sensitivity characteristics.

As shown in FIG. 4E, the photonic gas sensing system 410 may include a housing 420 that defines a chamber 424 for receiving and/or containing gas samples during sensing operations. The housing 420 may have a cylindrical shape with a cylindrical body configuration that provides a compact form factor suitable for integration into various gas monitoring systems. In some embodiments, the photonic gas sensing system 410 may include a tube positioned within the interior of the housing 420 to facilitate controlled gas flow through the chamber 424.

The housing 420 may include a gas inlet 426 configured to allow gas to enter the chamber 424 for analysis. The gas inlet 426 may be positioned to enable controlled introduction of gas samples into the chamber 424 where the gas samples may interact with the evanescent field of the silicon nitride waveguide 112. A gas outlet 428 may be configured to allow gas to exit the chamber 424 after sensing operations, enabling continuous flow of gas samples through the photonic gas sensing system 410 for real-time monitoring applications.

With continued reference to FIG. 4E, the photonic gas sensing system 410 may include a dual comb source 440 configured to generate a light beam 442 (input light beam) directed into the photonic gas sensor 400. The dual comb source 440 may comprise a dual frequency comb spectroscopy light source that provides optical signals with precise frequency characteristics suitable for enhanced gas sensing applications. The dual comb source 440 may be configured to generate dual frequency combs (e.g., two optical frequency combs) including a first optical frequency comb 442 (light beam 442) and second optical frequency comb 444 (light beam 444). The dual comb source may be configured to generate a first optical frequency comb 442 having a first repetition frequency and a second optical frequency comb 444 having a second repetition frequency that is different from the first repetition frequency. The dual comb source 440 may utilize different technological approaches to achieve the generation of synchronized pair of optical frequency combs with controlled repetition frequency differences. In some embodiments, the dual comb source may comprise two pulsed lasers that generate the pair of optical frequency combs with controlled repetition frequencies differences. In some embodiments, the pair of optical frequency combs may be characterized by a set of regularly spaced frequencies in its spectral content and short pulses in the time domain.

The input light beam 442 may be directed into the photonic gas sensor 400 through the light inlet 412, enabling optical coupling into the silicon nitride waveguide 112 through the light input coupler 124.

A photodetector 454, such as diode photodetector or other optical detectors, may be configured to receive light that is output from the photonic gas sensor 400 and generate a signal based on interaction of the light beam 442 with gas in the chamber 424. The photodetector 454 may receive optical signals, including output light beam 446 (e.g., resulting light beam after interaction of the input light beam 442 with the gas sample) that exit the photonic gas sensor 400 through the light outlet 414 and light beam 444 (e.g., the second optical frequency comb), where the optical signals may contain spectroscopic information about gas samples present in the chamber 424. As shown in FIG. 4E, in some embodiments, the optical signals may be combined to generate combined optical signal before being directed or detected by the photodetector 454. For example, the output optical frequency comb (e.g., output light beam 444) and the second optical frequency comb (e.g., light beam 444) from the pair of optical frequency combs may be combined to generate a combined optical frequency comb 448 (e.g., combined optical signal).

The photodetector 454 may convert the received optical signals into electrical signals for subsequent processing and analysis. As further shown in FIG. 4E, the photonic gas sensing system 410 may include a signal processor 456 configured to process the electrical signals from the photodetector 454 to determine a concentration of gas in the chamber 424. The signal processor 456 may analyze the electrical signals from the photodetector 454 to extract spectroscopic information that indicates the presence and concentration of target gases. In some embodiments, the signal processor 456 may perform signal conditioning, filtering, and analysis operations to enhance the accuracy of gas concentration measurements.

A data processor 458 may be configured to generate a gas sensing result 460 based on output from the signal processor 456. The data processor 458 may perform computational analysis of the processed signals to determine specific gas concentrations and generate output data suitable for display, logging, or transmission to external monitoring systems. The gas sensing result 460 may provide quantitative information about gas concentrations detected within the chamber 424.

The photonic gas sensing system 410 may include a control logic 452 configured to generate a heating control signal for the heating element 108 based on the signal from the photodetector 454. The control logic 452 may coordinate system operation by providing control signals to the dual comb source 440 and processing feedback from the photodetector 454 to maintain optimal sensing conditions. In some embodiments, the control logic 452 may regulate the operation of the heating element 108 to adjust a resonant frequency of the nitride waveguide 112 (or extended resonant ring waveguide 122 thereof) for precise frequency matching with the dual comb source 440. For example, in some embodiments, the control logic 452 may be configured adjust a resonant frequency of the nitride waveguide 112 (or extended resonant ring waveguide 122 thereof) for precise frequency matching with the repetition frequency of at least the first optical frequency comb 442.

The heating element 108 may adjust the resonant frequency through thermal expansion and thermal refractive index change of the silicon nitride waveguide 112, enabling precise matching with the repetition frequency of the dual comb source 440. The thermal tuning capability may maintain optimal resonance conditions that maximize the interaction between the light beam 442 and gas molecules in the chamber 424, enhancing the sensitivity of gas detection operations. The control logic 452 may provide feedback control to maintain stable resonance conditions during gas sensing operations.

As described in connection with the nitride waveguide 112 configuration, the spiral waveguide sections 122a may comprise four spiral waveguide symmetrically positioned within the extended resonant ring waveguide 122 to increase the effective optical path length to approximately 150mm. The extended optical path length created by the four spiral waveguide sections 122a may enable enhanced interaction between the light beam 442 and gas samples in the chamber 424, increasing the sensitivity of gas detection compared to conventional optical sensing systems.

The extended resonant ring waveguide 122 may have a ring diameter of approximately 3mm and may be configured to resonate at approximately 2GHz to match a dual frequency comb spectroscopy repetition frequency of the dual comb source 440. The combination of the compact ring diameter and extended optical path length may enable precise frequency matching while maintaining a compact form factor suitable for integration into the housing 420. The resonance at approximately 2GHz may provide optimal coupling with the dual comb source 440 for enhanced gas sensing performance.

The photonic gas sensing system 410 may enable continuous monitoring of gas concentrations through the coordinated operation of the optical, thermal, and signal processing components. Gas samples may flow through the chamber 424 via the gas inlet 426 and the gas outlet 428, allowing for real-time analysis of gas concentrations in industrial process streams, environmental monitoring applications, or healthcare diagnostic systems. The enhanced sensitivity provided by the extended optical path length and resonant enhancement may enable detection of trace gas concentrations that may be difficult to measure using conventional gas sensing technologies.

The dual comb source 440 may comprise a mode-locked laser system that generates optical frequency combs with precisely controlled repetition rates and spectral characteristics. The mode-locked laser system may include a master oscillator that produces a primary optical frequency comb and a secondary oscillator that generates a second optical frequency comb with a slightly different repetition rate. The frequency difference between the two combs may create a beat frequency pattern that enables high-resolution spectroscopic measurements with enhanced sensitivity compared to conventional single-frequency laser systems.

In some embodiments, the dual comb source 440 may operate with repetition frequencies selected to optimize coupling efficiency with the nitride waveguide structure. The repetition frequency of the dual comb source 440 may be stabilized using frequency locking techniques that maintain precise frequency control over extended operating periods. The frequency stabilization may include phase-locked loop circuits, frequency reference standards, or other stabilization methods that ensure consistent spectroscopic performance.

The light beam 442 (e.g., (e.g., optical frequency comb) generated by the dual comb source 440 may comprise a broadband optical spectrum that spans multiple absorption lines of target gas molecules. The broadband spectrum may enable simultaneous detection of multiple gas species within a single measurement cycle, providing comprehensive gas analysis capabilities for complex gas mixtures.

The photodetector 454 may comprise a high-speed photodiode, avalanche photodiode, or photomultiplier tube configured to detect optical signals across the spectral range of the dual comb source 440. In some embodiments, the photodetector 454 may include multiple detection elements arranged in an array configuration to enable simultaneous detection of different spectral components of the optical signal.

The signal processor 456 may include analog-to-digital conversion circuits that convert the electrical signals from the photodetector 454 into digital data suitable for computational analysis. The signal processor 456 may include digital signal processing algorithms that perform Fourier transforms, correlation analysis, or other mathematical operations to extract gas concentration information from the digitized signals. The data processor 458 may include machine learning algorithms or artificial intelligence systems that analyze spectroscopic patterns to identify and quantify gas concentrations with enhanced accuracy. The machine learning algorithms may be trained using reference gas samples with known concentrations to develop calibration models that account for environmental variations, temperature effects, and other factors that could affect measurement accuracy. The artificial intelligence systems may enable adaptive calibration that automatically adjusts measurement parameters to maintain optimal performance across different operating conditions. The control logic 452 may include feedback control algorithms that continuously monitor the resonance conditions of the ring resonator and automatically adjust the heating element 108 to maintain optimal frequency matching with the dual comb source 440. In some embodiments, the feedback control may utilize proportional-integral-derivative (PID) control algorithms or other control techniques that provide stable and responsive thermal tuning. The control logic 452 may include temperature sensors or optical monitoring systems that provide real-time feedback about the resonance conditions for closed-loop control operations.

The gas inlet 426 and gas outlet 428 may be configured with flow control elements that regulate the flow rate of gas samples through the chamber 424. The flow control elements may include mass flow controllers, pressure regulators, or valve systems that maintain consistent gas flow conditions for repeatable measurement results. The chamber 424 may be designed with specific volume and geometry characteristics that optimize the interaction between gas samples and the evanescent field of the silicon nitride waveguide 112. The chamber volume may be minimized to reduce response time while providing sufficient gas residence time for accurate concentration measurements. The housing may include temperature control features that maintain stable operating temperatures for the photonic gas sensor chip 100 and associated electronic components. The temperature control features may include thermal insulation, heat sinks, or active temperature regulation systems that prevent temperature variations from affecting measurement accuracy.

The photonic gas sensing system 410 may include calibration capabilities that enable periodic verification and adjustment of measurement accuracy using reference gas standards. The calibration capabilities may include automated calibration sequences that introduce known gas concentrations into the chamber 424 and compare measured results with expected values. The calibration data may be used to update measurement algorithms and maintain long-term accuracy of gas concentration measurements.

The enhanced sensitivity characteristics of the photonic gas sensing system 410 may enable detection of gas concentrations at parts-per-billion (ppb) or parts-per-trillion (ppt) levels, depending on the specific gas species and measurement conditions. The detection limits may be achieved through the combination of extended optical path length, resonant enhancement, and advanced signal processing techniques that maximize the signal-to-noise ratio of spectroscopic measurements. The enhanced sensitivity may enable applications in environmental monitoring, industrial safety, and medical diagnostics where detection of trace gas concentrations is critical for accurate analysis.

### Control System and Method of Operation

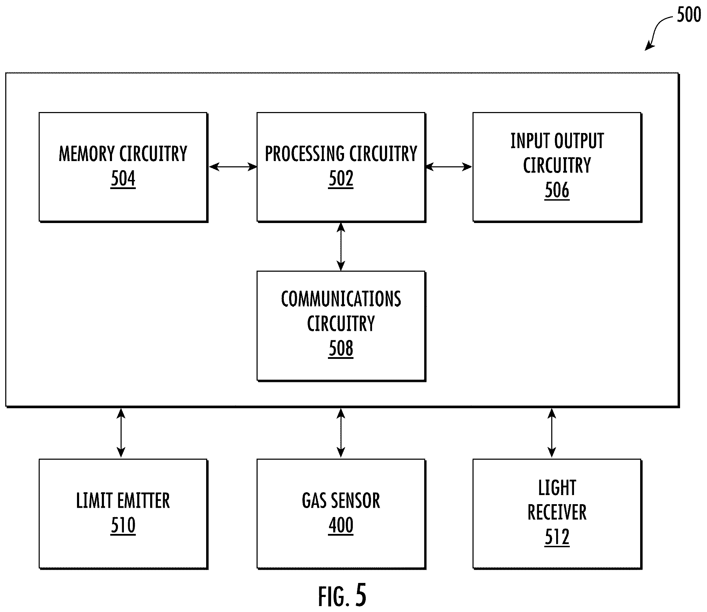

Referring to FIG. 5, a control device 500 may be configured to control operation of at least one of a light emitter 510, a light receiver 512, or a gas sensor 400 in a spectroscopy system for gas sensing applications. The control device 500 may provide centralized control and coordination of optical, thermal, and signal processing components to enable precise gas detection and analysis using dual frequency comb spectroscopy techniques. The control device 500 may include a control logic such as control logic 452 described above. The light emitter 510 may comprise dual comb source such as dual comb source 440 described above. In some embodiments, the light receiver 512 may comprise a photodetector such as photodetector 454 described above.

Although components are described with respect to functional limitations, it should be understood that at least some of the particular implementations necessarily include the use of particular computing hardware. It should also be understood that in some embodiments certain of the components described herein include similar or common hardware. For example, in some embodiments two sets of circuitries both leverage use of the same processor(s), memory(ies), circuitry(ies), and/or the like to perform their associated functions such that duplicate hardware is not required for each set of circuitries.

Processing circuitry 502 may be embodied in a number of different ways. In various embodiments, the use of the terms "processor" or "processing circuitry" should be understood to include a single core processor, a multi-core processor, multiple processors internal to the example device 500, and/or one or more remote or "cloud" processor(s) external to the example device 500. In some example embodiments, processing circuitry 502 may include one or more processing devices configured to perform independently. Alternatively, or additionally, processing circuitry 502 may include one or more processor(s) configured in tandem via a bus to enable independent execution of operations, instructions, pipelining, and/or multithreading.

In an example embodiment, the processing circuitry 502 may be configured to execute instructions stored in the memory circuitry 504 or otherwise accessible to the processor. Alternatively, or additionally, the processing circuitry 502 may be configured to execute hard-coded functionality. As such, whether configured by hardware or software methods, or by a combination thereof, processing circuitry 502 may represent an entity (e.g., physically embodied in circuitry) capable of performing operations according to embodiments of the present disclosure while configured accordingly. Alternatively, or additionally, processing circuitry 502 may be embodied as an executor of software instructions, and the instructions may specifically configure the processing circuitry 502 to perform the various algorithms embodied in one or more operations described herein when such instructions are executed. In some embodiments, the processing circuitry 502 includes hardware, software, firmware, and/or a combination thereof that performs one or more operations described herein.

In some embodiments, the processing circuitry 502 (and/or co-processor or any other processing circuitry assisting or otherwise associated with the processor) is/are in communication with the memory circuitry 504 via a bus for passing information among components of the example device 500.

Memory circuitry 504 may be non-transitory and may include, for example, one or more volatile and/or non-volatile memories. In some embodiments, the memory circuitry 504 includes or embodies an electronic storage device (e.g., a computer readable storage medium). In some embodiments, the memory circuitry 504 is configured to store information, data, content, applications, instructions, or the like, for enabling the example device 500 to carry out various operations and/or functions in accordance with example embodiments of the present disclosure.

Input/output circuitry 506 may be included in the example device 500. In some embodiments, input/output circuitry 506 may provide output to the user and/or receive input from a user. The input/output circuitry 506 may be in communication with the processing circuitry 502 to provide such functionality. The input/output circuitry 506 may comprise one or more user interface(s). In some embodiments, a user interface may include a display that comprises the interface(s) rendered as a web user interface, an application user interface, a user device, a backend system, or the like. In some embodiments, the input/output circuitry 506 also includes a keyboard, a mouse, a joystick, a touch screen, touch areas, soft keys a microphone, a speaker, or other input/output mechanisms. The processing circuitry 502 and/or input/output circuitry 506 may be configured to control one or more operations and/or functions of one or more user interface elements through computer program instructions (e.g., software and/or firmware) stored on a memory accessible to the processor (e.g., memory circuitry 504, and/or the like). In some embodiments, the input/output circuitry 506 includes or utilizes a user-facing application to provide input/output functionality to a computing device and/or other display associated with a user. In some embodiments, the input/output circuitry 506 one or more indicator lights or the like for providing a user notification (e.g., an alert or warning).

Communications circuitry 508 may be included in the example device 500. The communications circuitry 508 may include any means such as a device or circuitry embodied in either hardware or a combination of hardware and software that is configured to receive and/or transmit data from/to a network and/or any other device, circuitry, or module in communication with the example device 500. In some embodiments the communications circuitry 508 includes, for example, a network interface for enabling communications with a wired or wireless communications network. Additionally, or alternatively, the communications circuitry 508 may include one or more network interface card(s), antenna(s), bus(es), switch(es), router(s), modem(s), and supporting hardware, firmware, and/or software, or any other device suitable for enabling communications via one or more communications network(s). In some embodiments, the communications circuitry 508 may include circuitry for interacting with an antenna(s) and/or other hardware or software to cause transmission of signals via the antenna(s) and/or to handle receipt of signals received via the antenna(s). In some embodiments, the communications circuitry 508 enables transmission to and/or receipt of data from a user device, one or more sensors, and/or other external computing device(s) in communication with the example device 500.

In some embodiments, two or more of the sets of circuitries 502-508 are combinable. Alternatively, or additionally, one or more of the sets of circuitries 502-508 perform some or all of the operations and/or functionality described herein as being associated with another circuitry. In some embodiments, two or more of the sets of circuitries 502-508 are combined into a single module embodied in hardware, software, firmware, and/or a combination thereof.

While the description above provides an example device 500, it is noted that the scope of the present disclosure is not limited to the description above. In some examples, an example device 500 in accordance with the present disclosure may be in other forms. In some examples, an example device 500 may comprise one or more additional and/or alternative elements, and/or may be structured differently than that illustrated in FIG. 5.

A method of gas sensing using dual frequency comb spectroscopy may comprise directing a light beam from a dual frequency comb spectroscopy source into a silicon nitride waveguide comprising a ring resonator with spiral waveguide . The method may utilize the dual comb source 440 to generate the light beam 442 that may be coupled into the nitride waveguide 112 through the light input coupler 124. The silicon nitride waveguide may comprise the ring resonator with four spiral waveguide symmetrically positioned within the ring resonator to provide an effective optical path length of approximately 150mm.

The method may comprise adjusting a resonant frequency of the nitride waveguide (or extended resonant ring waveguide thereof) using a heating element 108 to lock the resonant frequency to a repetition frequency of the dual frequency comb spectroscopy source (e.g., repetition frequency of at least one optical frequency comb emitted by the dual comb source). The heating element 108 may adjust the resonant frequency through thermal expansion and thermal refractive index change of the silicon nitride waveguide 112, enabling precise matching with the repetition frequency of the dual comb source 440. The ring resonator may have a ring diameter of approximately 3mm and may resonate at approximately 2GHz to match the dual frequency comb spectroscopy repetition frequency.

The step of adjusting the resonant frequency may comprise applying electrical current to an aluminum heating element positioned beneath the silicon nitride waveguide. The aluminum heating element of the heating element 108 may receive electrical current through the heating current input pad 128 and the heating current output pad 130 to generate controlled thermal energy. The thermal expansion induced by the aluminum heating element may modify the effective optical path length of the nitride waveguide 112, thereby shifting the resonant frequency of the ring resonator to achieve precise frequency matching.

The method may comprise generating a heating control signal based on the detected changes to maintain resonance conditions for enhanced gas sensing sensitivity. The control logic 452 may generate the heating control signal for the heating element 108 based on signals from the photodetector 454 to maintain optimal resonance conditions. The heating control signal may provide feedback control that maintains stable resonance conditions during gas sensing operations, maximizing the interaction between the light beam 442 and gas molecules in the chamber 424 for enhanced sensitivity performance.

The method may comprise allowing or otherwise introducing a gas sample to interact with an evanescent field of the silicon nitride waveguide. The evanescent field may extend beyond the physical boundaries of the nitride waveguide 112 structure, enabling interaction with gas molecules present in the chamber 424 of the photonic gas sensing system 410. The gas sample may enter the chamber 424 through the gas inlet 426 and may contact the evanescent field through the gas window 404 that allows gas access to the photonic gas sensor chip 100.

The method may include detecting changes in the light beam caused by the interaction with the gas sample. The photodetector 454 may receive optical signals that exit the photonic gas sensor 400 through the light outlet 414, where the optical signals may contain spectroscopic information about gas samples present in the chamber 424. The changes in the light beam may result from absorption, scattering, or phase modifications caused by the interaction between the evanescent field and gas molecules in the sample.

The method may comprise processing the detected changes to determine a presence or concentration of a target gas in the gas sample. The signal processor 456 may analyze electrical signals from the photodetector 454 to extract spectroscopic information that indicates the presence and concentration of target gases. The data processor 458 may perform computational analysis of the processed signals to determine specific gas concentrations and generate the gas sensing result 460 that provides quantitative information about detected gas concentrations.

For example, the method may comprise combining output optical frequency comb corresponding to output light beam exiting the chamber and a second optical frequency comb from a pair of optical frequency combs (e.g., pair of optical frequency combs comprising the first optical frequency comb and second optical frequency comb) to generate a combined optical frequency comb; detecting, using an optical detector, the combined optical frequency comb; and processing the combined optical frequency comb to determine a presence or concentration of a target gas in the gas sample by performing one or more of signal processing or data processing based on the combined optical frequency comb.

The light receiver 512 may include multiple detection elements configured to simultaneously capture different spectral components of the optical signals exiting the photonic gas sensor 400. The multiple detection elements may comprise an array of photodiodes, each optimized for specific wavelength ranges corresponding to characteristic absorption features of different target gas molecules. In some embodiments, the detection array may include 4 to 16 individual photodiode elements arranged in a linear or two-dimensional configuration, enabling parallel detection of multiple gas species within a single measurement cycle.

The method of gas sensing using dual frequency comb spectroscopy may be implemented through a sequence of precisely timed operations that coordinate optical signal generation, gas sample introduction, and signal detection to achieve optimal measurement accuracy. The timing sequence may include initialization periods for thermal stabilization of the ring resonator, calibration cycles using reference gas standards, and measurement periods during which gas samples are analyzed.

The directing of the light beam from the dual frequency comb spectroscopy source may involve precise optical alignment procedures that ensure optimal coupling efficiency between the external dual comb source 440 and the light input coupler 124. The optical alignment may utilize precision positioning stages with sub-micrometer resolution to achieve coupling efficiencies exceeding 80% while maintaining stable optical coupling throughout temperature variations and mechanical vibrations. The alignment procedures may include automated feedback systems that monitor coupling efficiency and automatically adjust optical positioning to maintain optimal performance.

The spectroscopic information contained within the optical signals may include absorption line shapes, line widths, and line positions that provide quantitative information about gas concentrations and molecular properties. The absorption line analysis may utilize curve fitting algorithms that compare measured absorption profiles with reference spectra stored in spectroscopic databases. The curve fitting procedures may account for pressure broadening, temperature effects, and instrumental line shapes to achieve accurate gas concentration measurements with uncertainties of less than 1% for major gas components.

The processing of detected changes may involve real-time computational algorithms that perform spectral analysis within milliseconds of signal acquisition. The computational algorithms may include fast Fourier transform (FFT) operations, digital filtering, and statistical analysis procedures that extract gas concentration information from noisy measurement data. The real-time processing capability may enable continuous monitoring applications where immediate detection of gas concentration changes is required for safety or process control purposes.

Operations and processes described herein support combinations of means for performing the specified functions and combinations of operations for performing the specified functions. It will be understood that one or more operations, and combinations of operations, may be implemented by special purpose hardware-based computer systems which perform the specified functions, or combinations of special purpose hardware and computer instructions.

In some example embodiments, certain ones of the operations herein may be modified or further amplified as described below. Moreover, in some embodiments additional optional operations may also be included. It should be appreciated that each of the modifications, optional additions or amplifications described herein may be included with the operations herein either alone or in combination with any others among the features described herein.

The foregoing method and process descriptions are provided merely as illustrative examples and are not intended to require or imply that the steps of the various embodiments must be performed in the order presented. As will be appreciated by one of skill in the art the order of steps in the foregoing embodiments may be performed in any order. Words such as "thereafter," "then," "next," and similar words are not intended to limit the order of the steps; these words are simply used to guide the reader through the description of the methods. Further, any reference to claim elements in the singular, for example, using the articles "a," "an" or "the," is not to be construed as limiting the element to the singular and may, in some instances, be construed in the plural.

While various embodiments in accordance with the principles disclosed herein have been shown and described above, modifications thereof may be made by one skilled in the art without departing from the teachings of the disclosure. The embodiments described herein are representative only and are not intended to be limiting. Many variations, combinations, and modifications are possible and are within the scope of the disclosure. Alternative embodiments that result from combining, integrating, and/or omitting features of the embodiment(s) are also within the scope of the disclosure. Accordingly, the scope of protection is not limited by the description set out above, but is defined by the claims which follow, that scope including all equivalents of the subject matter of the claims. Each and every claim is incorporated as further disclosure into the specification and the claims are embodiment(s) of the present disclosure. Furthermore, any advantages and features described above may relate to specific embodiments but shall not limit the application of such issued claims to processes and structures accomplishing any or all of the above advantages or having any or all of the above features.

In addition, the section headings used herein are provided for consistency with the suggestions under 37 C.F.R. § 1.77 or to otherwise provide organizational cues. These headings shall not limit or characterize the disclosure set out in any claims that may issue from this disclosure. For instance, a description of a technology in the "Background" is not to be construed as an admission that certain technology is prior art to any disclosure in this disclosure. Neither is the "Summary" to be considered as a limiting characterization of the disclosure set forth in issued claims. Furthermore, any reference in this disclosure to "disclosure" or "embodiment" in the singular should not be used to argue that there is only a single point of novelty in this disclosure. Multiple embodiments of the present disclosure may be set forth according to the limitations of the multiple claims issuing from this disclosure, and such claims accordingly define the disclosure, and their equivalents, which are protected thereby. In all instances, the scope of the claims shall be considered on their own merits in light of this disclosure but should not be constrained by the headings set forth herein.

Also, systems, subsystems, apparatuses, techniques, and methods described and illustrated in the various embodiments as discrete or separate may be combined or integrated with other systems, modules, techniques, or methods without departing from the scope of the present disclosure. Other devices or components shown or discussed as coupled to, or in communication with, each other may be indirectly coupled through some intermediate device or component, whether electrically, mechanically, or otherwise. Other examples of changes, substitutions, and alterations are ascertainable by one skilled in the art and could be made without departing from the scope disclosed herein.

Many modifications and other embodiments of the disclosure set forth herein will come to mind to one skilled in the art to which these embodiments pertain having the benefit of teachings presented in the foregoing descriptions and the associated figures. Although the figures only show certain components of the apparatuses and systems described herein, various other components may be used in conjunction with the components and structures disclosed herein. Therefore, it is to be understood that the disclosure is not to be limited to the specific embodiments disclosed and that modifications and other embodiments are intended to be included within the scope of the appended claims. For example, the various elements or components may be combined, rearranged, or integrated in another system or certain features may be omitted or not implemented. Moreover, the steps in any method described above may not necessarily occur in the order depicted in the accompanying drawings, and in some embodiments one or more of the steps depicted may occur substantially simultaneously, or additional steps may be involved. Although specific terms are employed herein, they are used in a generic and descriptive sense only and not for purposes of limitation.

## Claims

1. A photonic gas sensor chip comprising:
a substrate;
a silicon oxide layer disposed on the substrate;
a heating element disposed on the silicon oxide layer;
a silicon oxide base disposed on the heating element; and
a silicon nitride waveguide disposed on the silicon oxide base, the silicon nitride waveguide comprising an extended resonant ring waveguide comprising a plurality of spiral waveguide sections.

2. The photonic gas sensor chip of claim 1 further comprising a silicon oxide cover disposed on the silicon nitride waveguide.

3. The photonic gas sensor chip of claim 1, wherein the plurality of spiral waveguide sections comprises four waveguide sections.

4. The photonic gas sensor chip of claim 1, wherein the plurality of spiral waveguide sections are arranged at regular angular intervals around a central axis of the extended resonant ring waveguide.

5. The photonic gas sensor chip of claim 1, wherein the silicon nitride waveguide further comprises:
an input waveguide configured to couple light into the extended resonant ring waveguide; and
an output waveguide configured to light from the extended resonant ring waveguide.

6. The photonic gas sensor chip of claim 5, wherein the extended resonant ring waveguide is positioned between the input waveguide and the output waveguide.

7. The photonic gas sensor chip of claim 5, wherein the silicon nitride waveguide further comprises:
a light input coupler configured to couple light into the input waveguide; and
a light output coupler configured to extract light from the output waveguide.

8. The photonic gas sensor chip of claim 7, wherein the light input coupler and the light output coupler each comprise periodic grating features.

9. The photonic gas sensor chip of claim 7, wherein each of the input waveguide and output waveguide is straight bus waveguide.

10. The photonic gas sensor chip of claim 1, wherein the extended resonant ring waveguide has a ring diameter of approximately 3mm and an effective optical path length of approximately 150mm.

11. The photonic gas sensor chip of claim 1, wherein the extended resonant ring waveguide is configured to resonate at approximately 2GHz to match a dual frequency comb spectroscopy repetition frequency.

12. The photonic gas sensor chip of claim 1, wherein the heating element is an aluminum heating element having a spiral pattern.

13. The photonic gas sensor chip of claim 1, wherein the heating element comprises:
a heating current input pad configured to receive electrical current from an external power source for supplying the heating element; and
a heating current output pad electrically connected to the heating current input pad.

14. A photonic gas sensing device comprising:
a housing defining a chamber;
a photonic gas sensor positioned within at least a portion of the housing, the photonic gas sensor comprising a silicon nitride waveguide having an extended resonant ring waveguide that extends an optical path length within the extended resonant ring waveguide;
a gas inlet configured for introducing gas into the chamber; and
a light inlet configured to receive light from an external light source.

15. A method of gas sensing using dual frequency comb spectroscopy, the method
comprising:
directing input light beam from a dual frequency comb into a silicon nitride waveguide positioned within a chamber, wherein the input light beam comprises a first optical frequency comb of a pair of optical frequency combs, wherein the silicon nitride waveguide comprises an extended resonant ring waveguide with spiral waveguide sections;
introducing a gas sample into the chamber, wherein the gas travels over the silicon nitride waveguide and interacts with an evanescent field of the silicon nitride waveguide;
combining output optical frequency comb corresponding to output light beam exiting the chamber and a second optical frequency comb from the pair of optical frequency combs to generate a combined optical frequency comb;
detecting, using an optical detector, the combined optical frequency comb; and
processing the combined optical frequency comb to determine a presence or concentration of a target gas in the gas sample by performing one or more of signal processing or data processing based on the combined optical frequency comb.
